# EUROPEAN PATENT SPECIFICATION

(11) **EP 1 601 336 B1**
(45) Date of publication and mention of the grant of the patent: **17.12.2008**
(21) Application number: 03765173.4
(22) Date of filing: 22.07.2003
(51) Int. Cl.: A61Q 11/00, A61K 8/02, A61K 8/04, A61K 8/19, A61K 8/25, A61K 8/31, A61K 8/33, A61K 9/14

(54) **AEROSOL DENTIFRICE FORMULATION**
ZAHNPUTZFORMULIERUNG IN AEROSOLFORM
FORMULATION DE DENTIFRICE SOUS FORME D'AEROSOL

(30) Priority: 23.07.2002 GB 0217078; 17.04.2003 GB 0308978
(43) Date of publication of application: 07.12.2005
(73) Proprietor: GLAXO GROUP LIMITED, Greenford, Middlesex UB6 ONN (GB)
(72) Inventor: FROST, Peter, Weybridge, Surrey KT13 0DE (GB)
(74) Representative: Reeves, Julie Frances
(86) International application number: PCT/GB2003/003150
(87) International publication number: WO 2004/009049

(56) References cited:
- AU-B- 523 336
- DE-A- 10 008 836
- GB-A- 1 015 917
- US-A- 2 995 521
- US-A- 3 629 398

## Description

This invention relates to dentifrice formulations, in particular to a dentifrice formulation that can be stored in a pressurised container from which it can be dispensed onto a toothbrush as a stable and controllable foam.

Dentifrices are commonly provided as a paste, i.e. a toothpaste, in a collapsible container from which they can be extruded. It is also known to provide a dentifrice formulation as a foam, e.g. as disclosed in DE-A-100 08 834, DE-A-100 08 836 and DE-A-100 08 837. Such formulations generally comprise a fluid mixture containing one or more abrasive, thickener, flavour together with a propellant, normally a liquefied gas with a boiling point below ambient temperature to drive the formulation out of its container and to expand to foam the formulation. The propellant in these prior art formulations will produce a post-foaming effect.

With this type of formulation it is particularly important to provide a foaming dentifrice that is both practical in use but also gives an immediate visual impact upon dispensing. Japanese Patent Application No.51-12593B discloses a dual phase oil-in-water emulsion formulation for a rapidly collapsing foam aerosol product. The formulation comprises a base agent and a mixed propellant comprising 70-95 wt% dimethylether (DME) and 30-5 wt% water insoluble liquified gas such as a flurocarbon, liquid petroleum, vinyl chloride and methyl chloride. This propellant system is important for products that require a rapid collapsing foam like nail polish or hair spray.

With aerosol dentifrice formulations it is very important to control the production and expansion of foam so that it can be controlled, particularly onto a small surface area toothbrush head. Also, the post-drooling effect on and around the actuator suffered by many aerosol products can lead to unattractive product that becomes unacceptable to use. It is important therefore that the product must not expand significantly further once dispensed onto a toothbrush or suffer from post-drooling effects. With this control and aesthetic appeal the product will be both pleasant and acceptable to the user.

Dentifrice formulations usually contain a high proportion of water and many of the more commonly used propellants, eg, hydrocarbon propellants, are not very miscible with water making the formulation of an acceptable foaming dentifrice product a challenge for those skilled in the art. As a result, many propellants will not provide acceptable foaming dentifrice formulations, particularly those formulations that are single phase water based.

Therefore it is an object of the present invention to provide a foaming dentifrice formulation with a suitable propellant system that produces a very stable foam and avoids all of the above mentioned problems.

The word foam in the text means a viscous liquid material that includes a large number of small bubbles of vapour of a propellant dispersed therein.

According to this invention an aerosol dentifrice formulation is provided comprising water, a particulate abrasive and a propellant, characterised in that the formulation contains 4-14 not % of a propellant which comprises a non-hydrocarbon being 2-8 wt.% of the formulation and a hydrocarbon propellant being 2-6 wt.% of the formulation.

These propellants are well known to those skilled in the art of aerosol formulation.

Suitable non-hydrocarbon propellants include dimethylether (DME), chlorofluorocarbon (CFC), hydrofluorocarbon (HFC), hydrochlorofluorocarbon (HCFC), nitrogen, carbon dioxide, nitrous oxide and compressed air. A preferred non-hydrocarbon propellant is DME. DME is useful as a propellant because soluble in water based products.

Suitable hydrocarbon propellants generate a range of pressures of ca.16-105 psi. Many propellants are known which can achieve this, suitably a commercial product "Butane 30" comprising a mixture of n-butane, i-propane and n-propane. Experiments have shown that when the dentifrice formulation comprises a mixture of hydrocarbon propellant such as n-butane and non-hydrocarbon propellant DME an optimised product is achieved that has a particularly stable foam without any significant expansion or collapsing of the foam.

Suitable liquified hydrocarbon propellants include one or more C₃ to C₅ hydrocarbon (HC) such as propane, n-butane or butane 22.

Typically a maximum total of 8 wt% propellant is used, more preferably 5 wt%. Preferably the dentifrice formulation of the present invention may comprise between 2-5 wt% of such a liquefied gas propellant.

The formulation is normally stored in a container provided with a release valve, under a pressure corresponding to the vapour pressure of the liquefied propellant at the storage temperature, and on opening the valve the formulation is expelled as a foam, e.g. onto a toothbrush head.

Typically the container may be provided with an actuator device by means of which the valve can be opened and the flow of formulation directed.

The abrasive typically comprises 1-10% by weight of the total mixture and has a particle size in the range of between 5-40 microns. Preferably the formulation comprises 9 wt% or less, e.g. 3-7 wt% abrasive, especially 4.5-6 wt%, typically ca. 5 wt%. The abrasive particle size is in the range of 5-40 microns, preferably 30 microns or less, more preferably 10 microns or less. Suitably a mixture of at least one less hard and at least one more hard particulate abrasive is used, typically in a proportion more hard: less hard in the range 1:1-5, suitably in the range 1:2.5-3.5. Suitably the abrasive material may be a silica or a combination of silicas. Less hard and more hard abrasives can also be called soft and hard abrasives and this will refer specifically to the hardness of the abrasive particle. Suitable silicas include those known as Zeodent 124™ (hard) and Zeodent 623™ (soft). The proportion and particle size of the abrasive are found to optimise the combination of suitability for flow of the formulation out through the valve and effective tooth cleaning.

The formulation preferably also contains one or more of the following.

One or more humectant, typically in a proportion of 25-75 wt%, preferably 45-55 wt%, especially 50±2 wt%. Humectants are added to protect the formulation from drying out and to provide consistency and protection against cold. Suitable humectants include sorbitol and glycerol. Suitably a mixture of sorbitol and glycerol may be used e.g. in a sorbitol:glycerol ratio in the range 1:1.5 - 1.5:1. Other humectants that may be used include xylitol, mannitol, 1,2-propylene glycol or mixtures of these polyols.

One or more slurrying/suspending agent, typically in a proportion of 1-5 wt%, preferably 2-3 wt%. A preferred slurrying agent is polyethylene glycol, e.g. of molecular weight in the range 200 - 800, typically ca. 300.

One or more surfactant. Typically a surfactant may be used as a foaming agent as foaming is one property of a surfactant. In this respect a surfactant is sometimes called a foaming agent. Suitable surfactants include anionic surfactants such as a sodium alkyl sulphate with 12-18 carbon atoms in the alkyl chain, such as sodium lauryl sulphate. Zwitterionic, ampholytic and non-ionic surfactants may also be used. A mixture of surfactants may be used. Suitably the surfactant may comprise 0.1-3.0 wt% of the formulation, preferably 1-2 wt%.

One or more thickening agent. Typically a thickening agent will add body to the foam. Typical thickening agents include hydroxypropylmethylcellulose (HPMC), hydroxyethylcellulose (HEC) and hydroxymethylcellulose (HMC) and the acrylic polymer Carbopol. Preferred thickening agents include xanthan gum which is a polysaccharide and/or a thickening silica, Zeodent 163. Typically the thickening agent may comprise 0.1-4.0 wt% of the formulation, typically 0.2-3.0 wt%. It is found that use of xanthan gum and Zeodent 163 can lead to a creamier foam with improved flow and texture characteristics.

One or more pH regulator, preferably to maintain the pH at 6.0-10.0, especially at ca. pH 8.0. Such a pH is found suitable to avoid corrosion of the tin plate, aluminum or containers that may be lacquered that are commonly used for containing such formulations. A suitable pH regulator is sodium hydroxide.

One or more other excipent such as a sweetener, colour, preservative, flavours, dyes, typically comprising up to ca. 2 wt% of the formulation.

Such compositions of the present invention may also comprise other active agents conventionally used in dentifrice compositions, for instance:
an antimicrobial agent, anti-plaque agent such as chlorhexidine or triclosan; anti-calculus agent such as a tetra- or a di-alkali metal pyrophosphate salt, or a mixture thereof, an alkali metal tripolyphosphate salt or an azacycloheptane diphosphonate salt; an anti-sensitivity agent that acts as a nerve depolariser, tubule occluder or mineralizer. These agents include e.g. strontium acetate, strontium chloride or a potassium salt such as potassium nitrate, potassium chloride or potassium citrate; remineralisation agent, a whitening agent such as tetra- or a di-alkali metal pyrophosphate or phosphate salts or peroxides, vitamins, fluoride, e.g. sodium fluoride, typically comprising up to ca. 0.5 wt% of the formulation. Such agents will be included at levels to provide the desired therapeutic effect.

Many other examples of materials of these types are known in the state of the art, e.g. in DE-A-100 08 837.

In order for the formulation to be a fluid mixture, the remainder of the formulation comprises water, typically the formulation comprises ca. 25-50 wt% water, preferably 30-40 wt% of the formulation.

A typical formulation according to this invention therefore comprises: one or more humectant 45-55 wt%, slurrying agent 2-3 wt%, surfactant 1-2 wt%, abrasives 3-7 wt%, preferably 3-5 wt %, thickening agent 0.2-0.5 wt%, flavour, active and sweetener 0-2 wt%, pH adjuster if necessary to provide pH of 8.5+/- 0.2, water 30-40 wt % preferably 35 +/- 1 wt%. This fluid formulation which is also known as the intermediate formulation is preferably charged into a metal or plastic container with a dispensing valve, at a proportion of 95-97 wt% with 3-5 wt% propellant providing a pressure of between 25-70, preferably 40-60 psi.

The preferred materials and their proportions described above also contribute to improved flow and handling of the formulation.

The following observations in the finished product were noted:

| | | | | | |
|---|---|---|---|---|---|
| N-butane | 0% | 2% | 2.5% | 3% | 5% |
| Dimethylether | 5% | 3% | 2.5% | 2% | 0% |
| Observation | Foam which collapses instantly | Stable foam, no expansion or collapse | Very slightly expanding foam | Slightly expanding foam | Very expanding foam |

A "very slightly expanding foam" can be defined as a foam that when dispensed onto a toothbrush head may show a small but insignificant expansion.

A typical process for making the formulation of this invention may involve the steps of:
- 1.: Adding a suitable quantity of water to a mixing vessel.
- 2.: Adding sweetener and active to the water and agitating until dissolved or suspended.
- 3.: Adding the humectant and agitating until homogeneous.
- 4.: Sieving the abrasive to break up any lumps. A 500 micron sieve is generally suitable.
- 5.: Slowly adding the abrasive to the mixture while mixing.
- 6.: Slurrying the thickening agent and slurrying agent and add to the mixture, agitate until homogeneous.
- 7.: Mixing, optionally transferring to a mixer.
- 8.: Slurrying the flavour, dye and foaming agent and adding to the mixture, mixing until homogeneous.
- 9.: Adjusting the pH and
- 10.: Mixing until homogeneous.

This fluid mixture may then be charged into suitable valved containers together with a suitable quantity of propellant.

The formulation may be used in a generally conventional manner involving opening the valve of the container to allow the internal pressure to expel the formulation onto a toothbrush. The invention also provides a valved container containing a formulation as described above.

The invention will now be described by way of examples.

### Example 1

A dentifice formulation was prepared having the following composition:

| **Function** | **Component** | **wt%** |
|---|---|---|
| Humectant | Sorbitol 70% non-crystallizing | 28.000 |
| Humectant | Glycerin | 22.00 |
| Suspending agent | PEG 6 | 2.500 |
| Foaming agent | Empicol 0303 30% solution | 5.000 |
| Sweetener | Sodium saccharin | 0.300 |
| Active | Sodium fluoride | 0.306 |
| Flavour | Flavour | 1.000 |
| Abrasive (hard) | Zeodent 124* | 1.330 |
| Abrasive (soft) | Zeodent 623* | 3.670 |
| Thickener | Xanthan (Keltrol F) | 0.250 |
| pH adjuster | 35% NaOH solution | 0.250 |
| Dye | Dye | 0.003 |
| Water | to | 100.000 |
| | | |
| Propellant | Butane | 2.000 |
| Propellant | DME | 3.000 |

| | | |
|---|---|---|
| *Zeodent 124 and Zeoent 623 are trade names of Huber Corporation, USA | | |

### Example 2

A dentifice formulation was prepared having the following composition:

| **Function** | **Component** | **wt%** |
|---|---|---|
| Humectant | Sorbitol 70% non-crystallizing | 28.000 |
| Humectant | Glycerin | 22.00 |
| Suspending agent | PEG 6 | 2.500 |
| Foaming agent | Empicol 0303 30% solution | 5.000 |
| Sweetener | Sodium saccharin | 0.300 |
| Active | Sodium fluoride | 0.306 |
| Flavour | Flavour | 1.000 |
| Abrasive (hard) | Zeodent 124 | 1.330 |
| Abrasive (soft) | Zeodent 623 | 3.670 |
| Thickener | Zeodent 163 | 3.000 |
| pH adjuster | 35% NaOH solution | 0.250 |
| Dye | Dye | 0.003 |
| Water | to | 100.000 |
| | | |
| Propellant | Butane | 3.000 |
| Propellant | DME | 2.000 |

| | | |
|---|---|---|
| *Zeodent 124 and Zeoent 623 and Zeodent 163 are trade names of Huber Corporation, USA | | |

### Example 3

A dentifice formulation was prepared having the following composition:

| **Function** | **Component** | **wt%** |
|---|---|---|
| Humectant | Sorbitol 70% non-crystallizing | 28.000 |
| Humectant | Glycerin | 22.00 |
| Suspending agent | PEG 6 | 2.500 |
| Foaming agent | Empicol 0303 solid | 1.500 |
| Sweetener | Sodium saccharin | 0.300 |
| Active | Sodium fluoride | 0.306 |
| Flavour | Flavour | 1.000 |
| Abrasive (hard) | Zeodent 124 | 1.330 |
| Abrasive (soft) | Zeodent 623 | 3.670 |
| Thickener | Zeodent 163 | 3.000 |
| Thickener | Xanthan (Keltrol F) | 0.250 |
| Dye | Dye | 0.003 |
| Water | to | 100.000 |
| | | |
| Propellant | Butane | 3.000 |
| Propellant | DME | 2.000 |

| | | |
|---|---|---|
| *Zeodent 124 and Zeoent 623 and Zeodent 163 are trade names of Huber Corporation, USA | | |

This fluid formulation was made by a process as described above, involving
- 1.: Adding a suitable quantity of water to a mixing vessel.
- 2.: Adding sweetener and active to the water, mixing until dissolved using a circular Heidolph paddle stirrer.
- 3.: Adding the glycerol and sorbitol to the batch, mixing until dissolved using a circular paddle stirrer on the Heidolph.
- 4.: Sieving the abrasive to break up any lumps. A 500 micron sieve is generally suitable.
- 5.: Slowly adding the abrasive to the mixture, mixing using a circular paddle stirrer on the Heidolph.
- 6.: Slurrying the thickening agent and slurrying agent and add to the mixture, agitate until homogeneous.
- 7.: Transferring to an Ultra Turrux mixer and mixing for 5 minutes.
- 8.: Slurrying the flavour and foaming agent and adding to the mixture, mixing until homogeneous with a circular paddle stirrer on the Heidolph.
- 9.: Adjusting the pH to pH 8 (+/- 0.5) using the NaOH.
- 10.: Mixing until homogeneous on the Heidolph.

This fluid mixture was charged into valved containers together with the propellant in a 95:5 or 97:3 w/w ratio.

## Claims

1. An aerosol dentifrice formulation comprising water, a particulate abrasive and a propellant, **characterised in that** the formulation contains 4-14wt % of a propellant which comprises a non-hydrocarbon being 2-8wt % of the formulation and a hydrocarbon propellant being 2-6wt % of the formulation.

2. An aerosol dentifrice according to claim 1 wherein the non-hydrocarbon propellant comprises dimethylether (DME), a chlorofluorocarbon (CFC), a hydrofluorocarbon (HFC) or a hydrochlorofluorocarbon (HCFC), nitrogen, carbon dioxide, nitrous oxide or compressed air.

3. An aerosol dentifrice according to claim 2 wherein the non-hydrocarbon propellant comprises dimethylether (DME).

4. An aerosol dentifrice according to claim 1 wherein the hydrocarbon propellant comprises one or more C₃ to C₅ hydrocarbon (HC) such as propane, n-butane or butane 22.

5. An aerosol dentifrice according to any one of the above claims wherein the particulate abrasive comprises 1-10% by weight of the mixture and has a particle size in the range 5-40 microns and comprises a combination of a more hard and a less hard abrasive.

6. An aerosol dentifrice according to claim 5, wherein the more hard and less hard abrasive is in a proportion of 1:1-5.

7. An aerosol dentifrice according to any one of the above claims, wherein the particulate abrasive is a silica.

8. An aerosol dentifrice according to claim 7, wherein the particle size of the abrasive is less than 30 microns.

9. An aerosol dentifrice according to claim 8, wherein the particle size of the abrasive is less than 10 microns.

10. An aerosol dentifrice according to any one of the above claims wherein the water comprises 25-50 wt% of the formulation.

11. An aerosol dentifrice according to claim 10, additionally comprising a humectant.

12. An aerosol dentifrice according to claim 11, additionally comprising a suspending agent.

13. An aerosol dentifrice according to claim 12, additionally comprising a surfactant.

14. An aerosol dentifrice according to any one of the above claims additionally comprising xanthan gum and Zeodent 163.

15. An aerosol dentifrice according to any one of the above claims comprising 45-55 wt% humectant, 0.1-4 wt% suspending agent, 1-5 wt% surfactant, 3-7 wt% abrasive, the remainder being water and 3-5 wt% propellant.

16. An aerosol dentifrice according to any one of the above claims comprising a valved container containing the formulation.

## Patentansprüche

1. Aerosole Zahnputzmittelformulierung, die Wasser, ein partikuläres Scheuermittel und ein Treibmittel umfaßt, **dadurch gekennzeichnet, daß** die Formulierung 4 bis 14 Gew.% eines Treibmittels enthält, das ein Nicht-Kohlenwasserstoff-Treibmittel, das 2 bis 8 Gew.% der Formulierung ausmacht, und ein Kohlenwasserstoff-Treibmittel, das 2 bis 6 Gew.% der Formulierung ausmacht, umfaßt.

2. Aerosoles Zahnputzmittel gemäß Anspruch 1, worin das Nicht-Kohlenwasserstoff-Treibmittel Dimethylether (DME), ein Chlorfluorkohlenstoff (CFC), ein Fluorkohlenwasserstoff (HFC) oder ein Chlorfluorkohlenwasserstoff (HCFC), Stickstoff, Kohlenstoffdioxid, die Stickstoffoxid oder Druckluft umfaßt.

3. Aerosoles Zahnputzmittel gemäß Anspruch 2, worin das Nicht-Kohlenwasserstoff-Treibmittel Dimethylether (DME) umfaßt.

4. Aerosoles Zahnputzmittel gemäß Anspruch 1, worin das Kohlenwasserstoff-Treibmittel ein oder mehrere C₃₋₅-Kohlenwasserstoffe (HC) wie Propan, n-Butan oder Butan 22 umfaßt.

5. Aerosolee Zahnputzmittel gemäß einem der vorstehenden Ansprüche, worin das partikuläre Scheuermittel 1 bis 10 Gew.% der Mischung umfaßt und eine Partikelgröße im Bereich von 5 bis 40 µm aufweist und eine Kombination eines härteren und eines weniger harten Scheuermittels umfaßt.

6. Aerosoles Zahnputzmittel gemäß Anspruch 5, worin das härtere und weniger harte Scheuermittel in einem Verhältnis von 1:1 bis 5 vorliegt.

7. Aerosoles Zahnputzmittel gemäß einem der vorstehenden Ansprüche, worin das partikuläre Scheuermittel Siliciumdioxid ist.

8. Aerosoles Zahnputzmittel gemäß Anspruch 7, worin die Partikelgröße des Scheuermittels weniger als 30 µm ist.

9. Aerosoles Zahnputzmittel gemäß Anspruch 8, worin die Partikelgröße des Scheuermittels weniger als 10 µm ist.

10. Aerosoles Zahnputzmittel gemäß einem der vorstehenden Ansprüche, worin das Wasser 25 bis 50 Gew.% der Formulierung umfaßt.

11. Aerosoles Zahnputzmittel gemäß Anspruch 10, das zusätzlich ein Befeuchtungsmittel umfaßt.

12. Aerosoles Zahnputzmittel gemäß Anspruch 11, das zusätzlich ein Suspendiermittel umfaßt.

13. Aerosoles Zahnputzmittel gemäß Anspruch 12, das zusätzlich ein Tensid umfaßt.

14. Aerosoles Zahnputzmittel gemäß einem der vorstehenden Ansprüche, das zusätzlich Xanthan und Zeodent 163 umfaßt.

15. Aerosoles Zahnputzmittel gemäß einem der vorstehenden Ansprüche, das 45 bis 55 Gew.% Befeuchtungsmittel, 0,1 bis 4 Gew.% Suspendiermittel, 1 bis 5 Gew.% Tensid, 3 bis 7 Gew.% Scheuermittel umfaßt, wobei der Rest Wasser und 3 bis 5 Gew.% Treibmittel ist.

16. Aerosoles Zahnputzmittel gemäß einem der vorstehenden Ansprüche, das einen mit einem Ventil versehenen Behälter umfaßt, der die Formulierung enthält.

## Revendications

1. Formulation de dentifrice sous forme d'aérosol, comprenant de l'eau, un abrasif particulaire et un agent propulseur, **caractérisée en ce que** la formulation contient 4 à 14 % en poids d'un agent propulseur qui comprend un composé non hydrocarbure qui constitue 2 à 8 % en poids de la formulation et un agent propulseur hydrocarbure constituant 2 à 6 % en poids de la formulation.

2. Dentifrice sous forme d'aérosol selon la revendication 1, dans lequel l'agent propulseur non hydrocarbure comprend le diméthyléther (DME), un chlorofluorocarbone (CFC), un hydrofluorocarbone (HFC) ou un hydrochlorofluorocarbone (HCFC), de l'azote, de l'oxyde de carbone, de l'oxyde nitreux ou de l'air comprimé.

3. Dentifrice sous forme d'aérosol selon la revendication 2, dans lequel l'agent propulseur non hydrocarbure comprend le diméthyléther (DME).

4. Dentifrice sous forme d'aérosol selon la revendication 1, dans lequel l'agent propulseur hydrocarbure comprend un ou plusieurs hydrocarbures en C₃ à C₅ (HC) tels que le propane, le n-butane ou le butane 22.

5. Dentifrice sous forme d'aérosol selon l'une quelconque des revendications ci-dessus, dans lequel l'abrasif particulaire comprend 1 à 10 % en poids du mélange et a une taille de particules de l'ordre de 5 à 40 microns et comprend une combinaison d'un abrasif plus dur et d'un abrasif moins dur.

6. Dentifrice sous forme d'aérosol selon la revendication 5, dans lequel l'abrasif plus dur et l'abrasif moins dur sont compris dans une proportion de 1:1-5.

7. Dentifrice sous forme d'aérosol selon l'une des revendications ci-dessus, dans lequel l'abrasif particulaire est une silice.

8. Dentifrice sous forme d'aérosol selon la revendication 7, dans lequel la taille de particule de l'abrasif est inférieure à 30 microns.

9. Dentifrice sous forme d'aérosol selon la revendication 8, dans lequel la taille de particule de l'abrasif est inférieure à 10 microns.

10. Dentifrice sous forme d'aérosol selon l'une des revendications ci-dessus, dans lequel l'eau comprend 25 à 50 % en poids de la formulation.

11. Dentifrice sous forme d'aérosol selon la revendication 10, comprenant en outre un humectant.

12. Dentifrice sous forme d'aérosol selon la revendication 11, comprenant en outre un agent de mise en suspension.

13. Dentifrice sous forme d'aérosol selon la revendication 12, comprenant en outre un tensioactif.

14. Dentifrice sous forme d'aérosol selon l'une quelconque des revendications ci-dessus, comprenant en outre de la gomme xanthane et du Zeodent 163.

15. Dentifrice sous forme d'aérosol selon l'une quelconque des revendications ci-dessus, comprenant 45 à 55 % en poids d'humectant, 0,1 à 4 % en poids d'agent de mise en suspension, 1 à 5 % en poids de tensioactif, 3 à 7 % en poids d'abrasif, le reste étant de l'eau et 3 à 5 % en poids d'agent propulseur.

16. Dentifrice sous forme d'aérosol selon l'une quelconque des revendications ci-dessus, comprenant un conteneur à valve contenant la formulation.
